# EUROPEAN PATENT APPLICATION

(11) **EP 2 438 911 A1**
(43) Date of publication of application: **11.04.2012**
(21) Application number: 10013440.2
(22) Date of filing: 08.10.2010
(51) Int. Cl.: A61K 9/20, A61K 31/4439, A61K 31/64, A61K 9/16

(54) **Pharmaceuticals compositions comprising sulphonylurea-class insulin secretagogue and polyethylene glycol castor oil**

(71) Applicant: LEK Pharmaceuticals d.d., 1526 Ljubljana (SI)
(72) Inventor: The designation of the inventor has not yet been filed
(74) Representative: Hodzar, Damjan

(57) **Abstract**

The present invention relates to the field of a pharmaceutical technology. More specifically, the present invention relates to a pharmaceutical composition comprising sulphonylurea-class insulin secretagogue and a surface active agent. Surface active agent obtainable by reacting castor oil or hydrogenated castor oil with ethylene oxide, preferably hydrogenated castor oil, substantially improves dissolution of the sulphonylurea-class insulin secretagogue active pharmaceutical ingredient and at the same time, when both formulated into a pharmaceutical composition, ensures satisfying or exceeding other parameters like for example stability, hardness, friability and handling of said pharmaceutical composition.

## Description

### Field of the invention

The present invention relates to the field of a pharmaceutical technology. More specifically, the present invention relates to a pharmaceutical composition comprising sulphonylurea-class insulin secretagogue and a surface active agent. Further, the present invention provides for a process and use of said pharmaceutical composition.

### Background of the invention

Sulphonylurea-class insulin secretagogue have proved to be a class of potent drugs for treating diabetes. Sulphonylurea-class insulin secretagogue lowers blood glucose primarily by stimulating the secretion of insulin from functional pancreatic beta cells. In this way they exert a long-term effect of reducing the blood glucose levels. In addition, extrapancreatic effects may also play a role in the activity of sulfonylureas. An efficacious sulphonylurea-class insulin secretagogue is glimepiride. This is supported by both preclinical and clinical studies demonstrating that glimepiride administration can lead to increased release of insulin by pancreatic beta cells. However, the characteristic low solubility of sulphonylurea-class active pharmaceutical ingredients poses great difficulty to providing pharmaceutical composition of suitable solubility or dissolution rate. In such case, when active pharmaceutical ingredient is not soluble or only sparingly soluble in neutral or acidic pH region, the dissolution is poor and a sufficient blood concentration of active pharmaceutical ingredient can not be obtained. Specifically for the sulphonylurea-class insulin secretagogue pharmaceutical ingredients the prior art describes various solutions to address the solubility problem.

WO 2005/041962 A1 discloses a solid preparation comprising an insulin sensitizer, which can among others be pioglitazone; an insulin sectetagogue like for example glimepiride, and a polyoxyethylene sorbitan fatty acid ester. Polysorbate 80 is exemplified as a preferred polyoxyethylene sorbitan fatty acid ester.

In the US 2007/0264331 A1 immediate release of glimepiride was achieved by putting glimepiride in a film coating, wherein sodium lauryl sulphate was added. In addition, the glimepiride was micronized.

It is indeed desired to provide a pharmaceutical composition comprising sulphonylurea-class insulin secretagogue as an active pharmaceutical ingredient that enables enhanced solubility and relatively fast dissolution of the otherwise insoluble or sparingly soluble active ingredient and thus allows the active ingredient to be better utilized when such pharmaceutical composition is administered to a patient. In addition, adequate or even superior dissolution of the sulphonylurea-class insulin secretagogue is preferably ensured together with satisfying or exceeding other parameters of pharmaceutical formulation like for example stability, hardness, friability and handling properties.

### Summary of the invention

First aspect of the present invention is a pharmaceutical composition comprising a sulphonylurea-class insulin secretagogue and a surface active agent, wherein the surface active agent is obtainable by reacting castor oil or hydrogenated castor oil with ethylene oxide.

Second aspect of the present invention is a process for preparing a pharmaceutical composition comprising a sulphonylurea-class insulin secretagogue and a surface active agent, wherein the surface active agent is obtainable by reacting castor oil with ethylene oxide, wherein the process comprises dissolving the surface active agent in a solvent, dispersing therein the sulphonylurea-class insulin secretagogue by mixing and removing the solvent.

Third aspect of the present invention is a pharmaceutical composition according to the first aspect for use as a medicine.

Yet another aspect of the present invention is a use of a surface active agent as defined in the first aspect for enhancing dissolution of a sulphonylurea-class insulin secretagogue, preferably glimepiride.

### Preferred embodiments of the invention and advantageous combinations thereof

The aspects, advantageous features and preferred embodiments of the present invention summarized in the following items, respectively alone or in combination, further contribute to solving the object of the invention:
(1) A pharmaceutical composition comprising a sulphonylurea-class insulin secretagogue and a surface active agent, wherein the surface active agent is obtainable by reacting castor oil or hydrogenated castor oil with ethylene oxide, preferably hydrogenated castor oil.
(2) The pharmaceutical composition according to item (1), wherein the surface active agent is obtainable by reacting 30 to 100 moles of ethylene oxide with 1 mole of castor oil, preferably 30 to 60 moles of ethylene oxide with 1 mole of castor oil, more preferably 40 to 45 moles of ethylene oxide with 1 mole of castor oil.
(3) The pharmaceutical composition according to item (1) or (2), wherein the surface active agent is polyoxyl 40 hydrogenated castor oil (Cremophor RH 40).
(4) The pharmaceutical composition according to any one of the items (1) to (3), wherein at least 50%, preferably at least 70%, more preferably at least 90%, particularly substantially all of the sulphonylurea-class insulin secretagogue in the composition is in contact with the surface active agent.
(5) The pharmaceutical composition according to any one of preceding items, wherein the weight ratio of the surface active agent to the sulphonylurea-class insulin secretagogue is from 1:20 to 10:1, preferably from 1:10 to 5:1, more preferably from 1:8 to 2:1, even more preferably from 1:4 to 1:1, most preferably from 1:2 to 1:1.
(6) The pharmaceutical composition according to any one of preceding items, further comprising a polymer selected from a group containing cellulose derivative, polyethylene glycol, polyvinylpyrrolidone, polyvinylacetate, polyvinylalcohol, polyester, polyamide, polyanhydride, polyorthoester, polycarbonate, poly(phosphoester), poly(phosphazene), poly(iminocarbonate), mixtures and copolymers thereof, preferably is selected from the group consisting of cellulose derivative, polyethylene glycol, polyvinylpyrrolidone, polyvinylacetate, polyvinylalcohol, mixtures and copolymers thereof, more preferably is selected from hydroxypropylcellulose and hydroxypropylmethylcellulose, wherein the polymer is in contact with the sulphonylurea-class insulin secretagogue and the surface active agent.
(7) The pharmaceutical composition according to any one of preceding items, further comprising a disintegrant and optionally other excipients, wherein said other excipients are water soluble.
(8) The pharmaceutical composition according to item (7), wherein the water soluble excipient is a hydrophilic diluent.
(9) The pharmaceutical composition according to item (8), wherein the hydrophilic diluent is any modified or unmodified monosaccharide, straight or branched oligosaccharide, straight or branched polysaccharide, or mixtures thereof, wherein the monosaccharide, oligosaccharide or polysaccharide are optionally additionally substituted; preferably the hydrophilic diluent is selected from the group consisting of lactose, starch, sucrose, mannitol, sorbitol and cellulose derivatives, more preferably the hydrophilic diluent is lactose.
(10) The pharmaceutical composition according any one of items (7) to (9), wherein the disintegrant is selected from the group consisting of sodium croscarmellose, sodium starch glycolate, crospovidone, starch, modified starch, low substituted hydroxypropyl cellulose, microcrystallilne celllouse, preferably the disintegrant is sodium croscarmellose.
(11) The pharmaceutical composition according to any one of the preceding items, wherein the sulphonylurea-class insulin secretagogue is glimepiride, tolbutaminde, glibenclamide, gliclazide, glycopyraminde, chlorpropamide, tolazamide, acetohexamide, glipizide, glybuzole, gliquidone, glyclopyramide, glixosepid, glimidine, glypinamide, glyhexamide, glybuthiazole or a salt and/or a mixture thereof; preferably is glimepiride, tolbutaminde, glibenclamide, gliclazide, glipizide, or a salt and/or a mixture thereof; more preferably is glimepiride.
(12) The pharmaceutical composition according to any one of the preceding items, further comprising an active pharmaceutical ingredient from a thiazolidinedione class, preferably pioglitazone, rosiglitazone, reglixane, netoglitazone, balaglitazone, rivoglitazone, tesaglitazar, ragaglitazar, muraglitazar or a salt and/or a mixture thereof; more preferably comprises pioglitazone, rosiglitazone or a salt and/or a mixture thereof; yet more preferably comprises pioglitazone or a salt thereof, particularly is pioglitazone hydrochloride.
(13) The pharmaceutical composition according to any one of the preceding items, further comprising another active pharmaceutical ingredient selected from the group consisting of insulin, biguanide compound, aldose reductase inhibitor, HMG-CoA reductase inhibitor, angiotensin converting enzyme inhibitor, angiotensin II antagonist, calcium antagonist and diuretic.
(14) The pharmaceutical composition according to any one of the preceding items, wherein the composition is a mixture, powder, granule, pellet, tablet, capsule, troche or combination thereof, preferably is a tablet, wherein each of the compositions is optionally film coated or mantle coated.
(15) The pharmaceutical composition according to item (14), wherein the tablet is a double or multilayer tablet, optionally film coated or mantle coated.
(16) The pharmaceutical composition according to item (15), wherein the tablet is a bilayer tablet comprising glimepiride in a first layer and pioglitazone, preferably pioglitazone hydrochloride, in a second layer.
(17) A process for preparing a pharmaceutical composition comprising a sulphonylurea-class insulin secretagogue and a surface active agent, wherein the surface active agent is obtainable by reacting castor oil with ethylene oxide, wherein the process comprises dissolving the surface active agent in a solvent, dispersing therein the sulphonylurea-class insulin secretagogue by mixing and removing the solvent, wherein mixing is preferably done by using high shear mixer.
(18) The process according to item (17), wherein the high shear mixer is a convection mixer, preferably ultraturax.
(19) The process according to item (17) or (18), wherein the solvent is removed in a granulation process.
(20) The process according to any one of the items (17) to (19), wherein a polymer according to item (6) is further added; and/or an excipient according to item any one of items (7) to (9) is further added; and/or a disintegrant according to item (10) is further added; and/or a compound according to item (12) is further added; and/or another active pharmaceutical ingredient according to item (13) is added; and/or the composition is compressed in a tablet, optionally a bilayer tablet.
(21) The process according to any one of the items (17) to (20), wherein the sulphonylurea-class insulin secretagogue is as defined in item (11).
(22) The process according to any one of the items (17) to (21), wherein the castor oil is hydrogenated.
(23) A pharmaceutical composition according to any one of the items (1) to (16) for use as a medicine, preferably for treating diabetes.
(24) Use of a pharmaceutical composition according to any one of the items (1) to (16) for the manufacture of a medicament, preferably for treating diabetes.
(25) Use of a surface active agent as defined in any one of the items (1) to (3) for enhancing dissolution of a sulphonylurea-class insulin secretagogue, preferably glimepiride.

### Brief description of the drawings

Fig. 1: Comparative dissolution study in phosphate buffer with pH 6.8 of glimepiride combined with various surface active agents, optionally together with lactose.
Fig. 2: Dissolution study in a phosphate buffer with pH 6.8 of glimepiride in amorphous, crystalline form, or together with different surface active agents or excipients, i.e. dissolution study of different compositions of example 2.

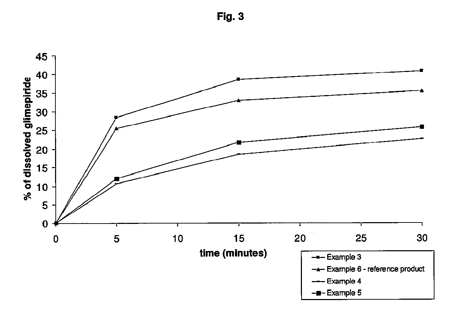

Fig. 3: Dissolution study of different pharmaceutical compositions of examples 3 to 6.

### Detailed description of the invention

A sulphonylurea-class insulin secretagogue in the context of the present invention means any active pharmaceutical ingredient that is an insulin secretion enhancer from the sulphonylurea-class of active pharmaceutical ingredients. For example, the sulphonylurea-class insulin secretagogue include glimepiride, tolbutaminde, glibenclamide, gliclazide, glycopyraminde, chlorpropamide, tolazamide, acetohexamide, glipizide, glybuzole, gliquidone, glyclopyramide, glixosepid, glimidine, glypinamide, glyhexamide, glybuthiazole or a salt and/or a mixture thereof. Preferably a sulphonylurea-class insulin secretagogue is preferably selected from glimepiride, tolbutaminde, glibenclamide, gliclazide, glipizide, or a salt and/or a mixture thereof; more preferably is glimepiride.

Sulphonylurea-based active pharmaceutical ingredients have very low and pH-dependent solubility. For example, in acidic medium glimepiride exhibits the solubility of less than 0.001 mg/mL at 37 °C. In media with pH around 7 the solubility of glimepiride is 0.001 mg/mL, of gliciazide is 0.05 mg/mL, of glipizide is 0.037 mg/mL and of glibenclamide is 0.06 mg/mL. At a pH 8, the solubility of glimepiride only slightly increases to 0,03 mg/mL. Thus, the sulphonylurea-class insulin secretagogues are insoluble or sparingly soluble. Being insoluble according to the present invention means that the solubility in water is lower than 10 mg/mL. Further, it may denote that the compound is substantially insoluble or completely insoluble in water, e.g. has a solubility in water of less than 0.05 mg/mL. Sparingly soluble means according to the present invention the solubility in water of between 10 mg/mL and 33 mg/mL. Solubility as meant herein is measured using USP apparatus 2 with agitation at 75 RPM. The dissolution medium is phosphate buffer maintained at 37 °C. Dissolution as meant herein is measured using HPLC.

Surprisingly it was discovered that the dissolution rate and the short term solubility, i.e. solubility in water or physiological environment in first 2 hours, preferably 4 hours, more preferably 8 hours, of the active pharmaceutical ingredients of a sulphonylurea-class insulin secretagogues can be significantly increased by simply combining a sulphonylurea-class insulin secretagogues with a surface active agent. The surface active agent used in the present invention means a detergent, surfactant or emulsifier like molecule that contains hydrophobic and hydrophilic structural moiety and is obtainable by reacting castor oil with ethylene oxide. Amounts of ethylene oxide can vary. For example the surface active agent can be obtained by reacting 30 to 100 moles of ethylene oxide with 1 mole of castor oil, preferably 30 to 60 moles of ethylene oxide with 1 mole of castor oil, more preferably 40 to 45 moles of ethylene oxide with 1 mole of castor oil. Castor oil is preferably hydrogenated. The main components of the surface active agent according to the present invention are fatty acid esters of glycerol polyethylene glycol and fatty acid esters of polyethylene glycol. They represent the hydrophobic part of the products. The hydrophilic part consists of polyethylene glycols and glycerol ethoxylate. The surface active agent can be further obtained by reacting 35 to 60 moles of ethylene oxide with 1 mole of hydrogenated castor oil glyceride, but is preferably obtained by reacting 35 to 45 moles of ethylene oxide with 1 mole of hydrogenated castor oil glyceride, more preferably 40 to 45 moles of ethylene oxide. The surface active agent is preferably polyoxyl 40 hydrogenated castor oil (Cremophor RH 40). As it was observed the surface active agent of the present invention can be used for enhancing dissolution of a sulphonylurea-class insulin secretagogue, preferably glimepiride. The surface active agent according to the present invention is particularly suitable because it does not affect a stability of the sulphonylurea-class active ingredient in the preparation. When compared to Polysorbate (polyoxyethylene sorbitan fatty acid ester), which is known to generate formaldehyde when exposed to oxygen (air) or light and thus may adversely affect the stability of the pharmaceutical active ingredient, no similar issues were observed with the surface active agent according to the present invention. Polyoxyethylene sorbitan fatty acid esters form peroxides at elevated temperatures (for example 40°C, 50°C or 60°C) in the presence of air. On the contrary, aqueous solution of polyoxyl castor oil can be sterilized by autoclaving for 30 minutes at 120°C. In this process, a product may acquire a deeper colour but this has no significance for product stability.

In a preferred embodiment, the pharmaceutical composition is prepared to have at least 50%, preferably at least 70%, more preferably at least 90%, particularly substantially all of the sulphonylurea-class insulin secretagogue in the composition in contact with the surface active agent. The close contact of the sulphonylurea-class active pharmaceutical ingredient with the surface active agent allows the surface agent to influence the microenvironment of the active pharmaceutical ingredient and the result is an advanced dissolution of said active pharmaceutical ingredient. Preparation procedures that yield a dispersed active pharmaceutical ingredient in the surface active agent result in a pharmaceutical composition with desired dissolution properties. In a preferred embodiment, the pharmaceutical composition further comprises a polymer, which can be any polymer that is suitable for binding, for film coating or for providing a tablet. The polymer is preferably in contact with a sulphonylurea-class insulin secretagogue and a surface active agent. The polymer can be selected from a group containing cellulose derivative, polyethylene glycol, polyvinylpyrrolidone, polyvinylacetate, polyvinylalcohol, polyester, polyamide, polyanhydride, polyorthoester, polycarbonate, poly(phosphoester), poly(phosphazene), poly(iminocarbonate), mixtures and copolymers thereof. Preferably the polymer is selected from the group consisting of cellulose derivative, polyethylene glycol, polyvinylpyrrolidone, polyvinylacetate, polyvinylalcohol, mixtures and copolymers thereof, more preferably is selected from hydroxypropylcellulose and hydroxypropylmethylcellulose. The polymer that is in contact with the sulphonylurea-class insulin secretagogue and the surface active agent binds both constituents and forces them to be in contact. The weight ratio of the surface active agent to the sulphonylurea-class insulin secretagogue in the pharmaceutical composition can be from 1:20 to 10:1, preferably from 1:10 to 5:1, more preferably from 1:8 to 2:1, even more preferably from 1:4 to 1:1. Most preferably the ratio of aforementioned constituents is from 1:2 to 1:1.

To attain preferred result in terms of improved dissolution, the pharmaceutical composition can further comprise a disintegrant and optionally other excipients, wherein said other excipients are water soluble. Preferably, all excipients, including the disintegrant, are water soluble. Such composition, when placed in water (or physiological environment), accepts water faster, preferably disintegrates faster and thus allows a faster onset of a sulphonylurea-class insulin secretagogue dissolution. The water soluble excipient that can be used in the pharmaceutical composition of the present invention is for example a hydrophilic diluent. The hydrophilic diluent can be any soluble or not soluble hydrophilic compound, optionally mixed with another at least one pharmaceutically acceptable excipient, present in a form of molecules, particles, powders, granules, beads, tablet cores, or the like, and can be for example any modified or unmodified monosaccharide, straight or branched oligosaccharide, straight or branched polysaccharide or mixtures thereof, wherein the monosaccharide, oligosaccharide or polysaccharide are optionally additionally substituted. The comprehension of the monosaccharide, oligosaccharide or polysaccharide is completely in the purview of the skilled person. For example, the hydrophilic diluent can be lactose, starch, sucrose, mannitol, sorbitol or cellulose derivatives. According to the preferred embodiment of the present invention lactose is used as a hydrophilic diluent. The hydrophilic diluent is preferably in the form of particles, thereby providing a favourable surface area for depositing a blend of the at least one sulphonylurea-class insulin secretagogue and the surface active agent according to the present invention in order to provide large surface area, faster wettabilitiy and thereby enhanced solubility in water. A preferred particle size of a particulate hydrophilic diluent is d0.9 < 150µm, more preferable d0.9 is < 100µm. Adding a disintegrant or preferably a superdisintegrant can assist in achieving desired dissolution rate of the active pharmaceutical ingredient of the sulphonylurea-class. The disintegrant can be for example sodium croscarmellose, sodium starch glycolate, crospovidone, starch, modified starch, low substituted hydroxypropyl cellulose or microcrystallilne celllouse. Preferably the disintegrant is sodium croscarmellose.

The pharmaceutical composition according to the present invention can comprise in addition to a sulphonylurea-class secretagogue an active pharmaceutical ingredient from a thiazolidinedione class. Thiazolidinedione class active pharmaceutical ingredients act by decreasing insulin resistance. Pharmacological studies indicate that they improve sensitivity to insulin in muscle and adipose tissue and inhibits hepatic gluconeogenesis. Also, they are a potent and highly selective agonist for peroxisome proliferators-activated receptor gamma. This receptors are found in tissues important for insulin action such as adipose tissue, skeletal muscle and liver. Activation of peroxisome proliferators-activated receptor gamma modulates a transcription of a number of insulin responsive genes involved in the control of glucose and lipid metabolism. Specific examples of thiazolidinedione class compounds are pioglitazone, rosiglitazone, reglixane, netoglitazone, balaglitazone, rivoglitazone, tesaglitazar, ragaglitazar, muraglitazar or a salt and/or a mixture thereof. Preferably used in the composition of the present invention are pioglitazone, rosiglitazone or a salt and/or a mixture thereof; yet more preferably pioglitazone or a salt thereof, particularly pioglitazone hydrochloride. Preferably the thiazolidinedione class compound is formulated in the pharmaceutical composition in a compartment different from where the sulphonylurea-class pharmaceutical ingredient is. In a similar manner, another active pharmaceutical ingredient like for example insulin, biguanide compound (e.g. phenformin, buformin or metformin), fibrate compound (e.g. bezafibrate, ciprofibrate, clofibrate, gemfibrozil or fenofibrate), aldose reductase inhibitor (e.g. tolrestat, epalrestat, zenarestat, minalrestat or fidarestat), HMG-CoA reductase inhibitor (e.g. atorvastatin, cerivastatin, fluvastatin, lovastatin, mevastatin, pitavastatin, pravastatin, rosuvastatin or simvastatin), angiotensin converting enzyme inhibitor (e.g. captopril, enalapril or delapril), angiotensin II antagonist (e.g. candesartan cilexetil, olmesartan medoxomil, losartan, valsartan, irbesartan, telmisartan or eprosartan), calcium antagonist (e.g. amlodipine, nifedipine, nicardipine or efonidipine) and diuretic (e.g. hydrochlorothiazide), can be added to the pharmaceutical composition of the present invention. Any combination of the sulphonylurea-class secretagogue with one or more aforementioned active pharmaceutical ingredients is contemplated by the present invention.

Aforementioned active pharmaceutical ingredients, including the sulphonylurea-class secretagogues, if appropriate, may be in the form of a salt. Such a salt may be a pharmacologically acceptable salt, such as a salt of organic or inorganic acid or base, or as a salt with amino acid.

The pharmaceutical compositions of the present invention, both as a product as such or during its production process, can be supplemented with additional excipients. Suitable excipients are any conventionally used pharmaceutically acceptable excipients. For example, the excipients are binder (polyvinylpyrrolidone, hydroxypropyl cellulose, hypromellose etc.), a lubricant (magnesium stearate, stearic acid, silicium dioxide, etc.), pH adjusting agent ( citrate, phosphate, acetate, amino acid salts, etc.), fragrant (menthol, peppermint oil, etc.), flavour (aspartame, saccharin sodium, etc.), colorant (titanium dioxide, etc.), a sweetener (saccharine, etc.), stabilizer (tocopherol, cyclodextrin, etc.) or coating polymers (hydroxypropylmethylcellulose, etc.), vehicles (water, organic solvents, etc.). Further, the pharmaceutical composition according to the present embodiment can be formulated in a mixture, powder, granule, pellet, tablet, capsule, troche or combination thereof, wherein the obtained formulations is optionally film coated or mantle coated. Preferably the pharmaceutical composition is a tablet, more preferably a double layer or multi layer tablet, particularly is a double layer tablet. Special designed tablets as for example sublingual, orally disintegrating or buccal tablet can also be prepared by the composition of the present invention.

The pharmaceutical composition according to the present invention is prepared by dissolving the surface active agent in a solvent and dispersing therein a sulphonylurea-class insulin secretagogue mixing, preferably by using a high shear mixer. The solvent used can be water, C1 to C4 alcohol, acetone or the like. The solvent can be further a mixture of various solvents. Preferably, the solvent is water. Thoroughly and uniformly dispersing the sulphonylurea-class insulin secretagogue in the solution of the surface active agent enhances the dissolution of the sulphonylurea-class active ingredient in the finalized composition. Thus, it is preferable to disperse the active ingredient by using a high shear convection mixer like for example Ultraturax. The obtained dispersion already assists in better utilization of otherwise sparingly soluble or insoluble sulphonylurea-class insulin secretagogue. Such dispersion can serve as a basis in all subsequent formulation steps. For example, the dispersion is used in a granulation process on its own, where a simple binary mixture of a sulphonylurea-class insulin secretagogue and the surface active agent, preferably in a form of a granule, is obtained. This can be achieved for example by a high shear granulation or fluid bed granulation. In the alternative, it can be applied to an excipient in a particulate form or a mixture thereof by for example spray drying or fluid bed granulation techniques. In any event, the solvent is at least partly, preferably substantially removed from the dispersion. The dispersion can be supplemented with a polymer, which normally assist in either obtaining stable aggregates or stable granules; or promotes depositing a binary blend of the sulphonylurea-class active ingredient and surface active agent on the particle surface of the excipient. The polymer and the excipient used in the process are as defined above. Further excipient or other active pharmaceutical ingredient as exemplified above may be added. One option is to add them to the starting dispersion or preferably extragranularly. (26) Further steps towards preparing a mixture, powder, granule, pellet, tablet, capsule, troche or combination thereof will be immediately evident to the person skilled in the art. For example, the pharmaceutical composition according to the present invention is filled in the hard gelatine or hypromellose capsules. A sulphonylurea-class insulin secretagogue and optionally other pharmaceutical ingredient, preferably of a thiazolidinedione class, can be included in the same granules or in separate granules or only one of active ingredients can be granulated. Double- or multilayer- tablet press may be employed to prepare double- or multi-layered tablets. Also, tablet press can be used to obtain mantle coating. Film coating, on the other hand, can be prepared by dissolving or dispersing the pharmaceutical composition in a suitable vehicle for film coating and depositing the vehicle on the cores. Various spheronization or extrusion techniques can be employed to prepare pellets. In the preferred embodiment the pharmaceutical composition in a form of a tablet is obtained, wherein the tables is a bilayer tablet comprising glimepiride in a first layer and pioglitazone, preferably pioglitazone hydrochloride, in a second layer.

In the preferred embodiment, the pharmaceutical composition is in a tablet. We observed that adding surface active agent obtainable by reacting castor oil with ethylene oxide, i.e. PEG castor oil or glycerol-polyethylene glycol oxystearate, preferably PEG hydrogenated castor oil; to the pharmaceutical composition does not cause the finished tablet to have unsatisfactory hardness. General routines for preparing tablets can be used. For example, when the tablets contain pharmaceutical composition comprising a sulphonylurea-class insulin secretagogue and the surface active agent as one of the layers in a double-layered tablet, the layer or tablet can be compressed by using bilayer rotary tablet press Piccola DC (Riva) employing oval punches (13.25 x 6.6 mm). Tabletting pressure can be for example in the range of about 7-20 kN, preferably 9-15 kN. Tablet hardness obtained in one embodiment is in the range of 70-120 N.

Yet more preferred embodiment of the present invention is a bi-layer tablet comprising:
a) a first layer comprising 0.5 - 8%, preferably 1.5 - 3%, of glimepiride; 0.1 - 8%, preferably 0.7 - 1.5%, of the surface active agent as defined above; 0.1 - 4%, preferably 1 - 2% of a polymer; 10 - 90%, preferably 40 - 60%, of a hydrophilic diluent, 0.5 - 20%, preferably 1 - 7%, of a disintegrant;
b) a second layer comprising at least one active pharmaceutical ingredient of the thiazolidinone class, preferably pioglitazone or rosiglitazone, more preferably pioglitazone, particularly pioglitazone hydrochlorothiazide,
wherein each % denote a weight percent calculated based on a weight of the first layer.

The pharmaceutical composition according to the present invention can be used as a medicine or for the manufacture of a medicament. The pharmaceutical composition is particularly useful for preventing and/or treating diabetes, hyperlipemia, impaired glucose tolerance, diabetic complications (e.g. nephrophaty, retinopathy), obesity. The administration route and dose regime of the pharmaceutical composition according to the present invention will be apparent to the skilled person. Generally, they depend on the active pharmaceutical ingredients included in the pharmaceutical composition, symptoms, indication, and/or patient. For example, the pharmaceutical composition of the present invention may comprise from 1 to 8 mg of glimepiride and from 1 to 45 mg of at least one active pharmaceutical ingredient of the thiazolidinone class. Preferably, the pharmaceutical composition comprises 2 mg of glimepiride and 30 mg of pioglitazone, 4 mg of glimepiride and 30 mg of pioglitazone or 4 mg of glimepiride and 45 mg of pioglitazone. The pharmaceutical composition of the present invention can be administered for example once or twice daily, again depending on the factors like the active pharmaceutical ingredients included in the pharmaceutical composition, symptoms, indication, and/or patient.

The following examples are merely illustrative of the present invention and they should not be considered as limiting the scope of the invention in any way, as these examples, modifications and other equivalents thereof will become apparent to those versed in the art in the light of the present disclosure, and the accompanying claims.

### Example 1

Samples 1 to 3 were prepared in the following manner:
Sample 1: Physical mixture of glimepiride and Texapon (sodium lauryl sulfate) 9 % m/m
Sample 2: Granulate comprising of glimepiride, Polysorbate and lactose (4/1/95 % m/m)
Sample 3: Granulate comprising of glimepiride, Cremophor RH 40 and lactose (4/1/95 % m/m)

Dissolution of the samples was tested under conditions: dissolution apparatus, paddle, 75 rpm, 37 °C, phosphate buffer pH=6,8 (900 mL). The samples were taken in various time points and HPLC measurements undertaken. The HPLC conditions were as follows. Waters system equipped with a C18, 3,5 µm, 150 x 4,6 mm column (XBridge) which was maintained in a column oven at 30 °C. The mobile phase A consisted of a mixture of a phosphate buffer of pH 2,5 and acetonitrile in ratio 72 : 28 (VN). The mobile phase B consisted of a mixture of a phosphate buffer of pH 2,5 and acetonitrile in ratio 30 : 70 (VN). The flow rate was 1.5 ml/min and the detection wavelength 230 nm.
Gradient

| time (min) | % A |
|---|---|
| 0 | 100 |
| 2 | 0 |
| 5 | 0 |
| 5,5 | 100 |

Solvent = mixture of water and acetonitrile = 200 : 800 (VN)
Stock solution was prepared in concentration of 0,02 mg/mL

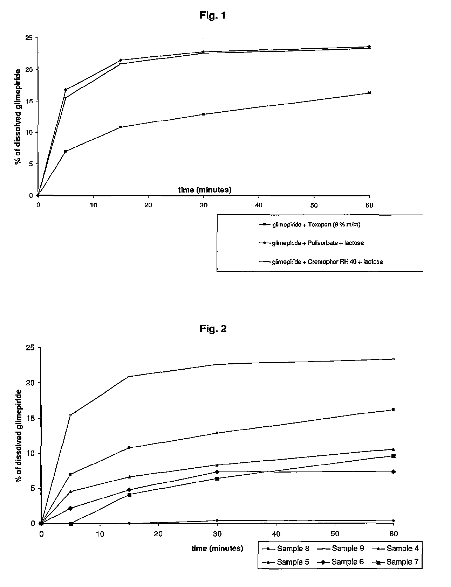

Results of the measurements are depicted on Figure 1. It can be concluded that glimepiride as a member of the sulphonylurea-class insulin secretagogues dissolves at a comparable rate when combined with polysorbate 80 or Cremophor 40 (polyoxyl 40 hydrogenated castor oil) together with lactose as a hydrophilic diluent. Yet, polysorbate 80 and Cremophor 40 achieve a superior dissolution of glimepiride when compared with sodium lauryl sulphate (Texapon).

### Example 2

Samples 4 to 9 were prepared in the following manner:
Sample 4: Crystalline glimepiride
Sample 5: Amorphous glimepiride
Sample 6: Physical mixture of glimepiride and Klucel EXF (hydroxypropyl cellulose) 33 % w/w
Sample 7: Physical mixture of glimepiride and PVP K 25 (polyvinylpyrrolidone) - 33 % w/w
Sample 8 (as sample 1): Physical mixture of glimepiride and Texapon (sodium lauryl sulfate) -9% w/w
Sample 9 (as sample 3): Granulate comprising of glimepiride, Cremophor RH 40 and lactose (4/1/95 % w/w)
Dissolution of the samples was tested under the same conditions as in example 1. Results are presented in Figure 2.

### Example 3

Aditionally, bilayer tablets contaning pioglitazone and glimepiride were produced on laboratory scale. The following tables were prepared:
Composition:

| | Per tablet (mg) |
|---|---|
| **Glimepiride layer** | |
| Glimepiride | 4.00 |
| Hydroxypropyl cellulose | 0.50 |
| Cremophor RH40 | 2.00 |
| Demineralised water* | 95.00 |
| Lactose monohydrate | 103.50 |
| Hydroxypropyl cellulose - powdered | 4.00 |
| Sodium croscarmellose | 11.40 |
| Microcrystalline cellulose | 54.00 |
| Magnesium stearate | 0.60 |
| | |
| **Pioglitazone layer** | |
| Pioglitazone hydrochloride | 33.07 |
| Lactose monohydrate | 29.67 |
| Sodium croscarmellose | 2.40 |
| Hydroxypropyl cellulose | 1.26 |
| Demineralised water* | 80.00 |
| Microcrystalline cellulose | 48.00 |
| Hydroxypropyl cellulose - powdered | 4.80 |
| Magnesium stearate | 0.80 |
| | |
| | Per tablet (mg) |
| Glimepriride layer | 180.00 |
| Pioglitazone layer | 120.00 |
| **Tablet mass** | 300.00 |

| | |
|---|---|
| *Not present in tablet - removed during processing | |

Cremophor RH 40 was dissolved in 2/3 demineralised water, glimepiride was added and dispersed using high shear convection mixer. Separately hydroxypropyl cellulose in dissolved in 1/3 demineralised water was added to dispersion of the active pharmaceutical ingredient. Thus prepared granulation liquid was sprayed onto lactose, hydoxypropyl cellulose-powdered and half of sodium corscarmellose mixture in fluid bed granulator. Microcrystalline cellulose and another half of sodium croscaremllose were added to obtained dry granulate and mixed in double cone mixer, finally magnesium stearate was added and mixed for a short period of time. Batch size was 1260 g.

Hydroxypropyl cellulose was dissolved in demineralised water and sprayed onto pioglitazone hydrochloride, lactose and half of sodium corscarmellose mixture in fluid bed granulator. Dry granulate was mixed with microcrystalline cellulose, powdered hydroxypropyl cellulose and other half of sodium croscaremllose in double cone mixer. Finally magnesium stearate was added and mixed for a short period of time. Batch size was 1800 g.

Thus prepared granulates were compressed into bilayer tablets using rotary tabletting machine.

### Example 4

Tablets containing glimepiride layer without a surface active agent were prepared having the following composition.
Composition:

| | Per tablet (mg) |
|---|---|
| **Glimepiride layer** | |
| Glimepiride | 4.00 |
| Hydroxypropyl cellulose | 1.50 |
| Demineralised water* | 140.00 |
| Lactose monohydrate | 154.80 |
| Hydroxypropyl cellulose - powdered | 4.40 |
| Sodium croscarmellose | 16.40 |
| Microcrystalline cellulose | 78.00 |
| Magnesium stearate | 0.90 |
| | |

| | Per tablet (mg) |
|---|---|
| Glimepriride layer (Example 4) | 260.00 |
| Pioglitazone layer (Example 3) | 120.00 |
| **Tablet mass** | 380.00 |

| | |
|---|---|
| *Not present in tablet - removed during processing | |

Hydroxypropyl cellulose was dissolved in demineralised water, glimepiride was added and dispersed using high shear convection mixer. Thus prepared granulation liquid was sprayed onto lactose, hydoxypropyl cellulose-powdered and half of sodium corscarmellose mixture in fluid bed granulator. Microcrystalline cellulose and the other half of sodium croscaremllose were added to obtained dry granulate and mixed in double cone mixer, finally magnesium stearate was added and mixed for a short period of time. Batch size was 1560 g.

Thus prepared granulate and pioglitazone granulate (Example 3) were compresed into bilayer tablets using rotary tabletting machine.

### Example 5

Tablets comprising glimepiride layer and sodium lauryl sulphate were prepared with the following composition.
Composition:

| | Per tablet (mg) |
|---|---|
| **Glimepiride layer** | |
| Glimepiride | 4.00 |
| Sodium lauryl sulphates | 1.00 |
| Hydroxypropyl cellulose | 4.50 |
| Demineralised water* | 95.00 |
| Lactose monohydrate | 104.90 |
| Sodium croscarmellose | 11.40 |
| Microcrystalline cellulose | 54.00 |
| Magnesium stearate | 0.60 |
| | |

| | Per tablet (mg) |
|---|---|
| Glimepriride layer (Example 5) | 180.40 |
| Pioglitazone layer (Example 3) | 120.00 |
| **Tablet mass** | 300.40 |

| | |
|---|---|
| *Not present in tablet - removed during processing | |

Sodium lauryl sulphate was dissolved in 2/3 demineralised water, glimepiride was added and dispersed using high shear convection mixer. Separately, hydroxypropyl cellulose was dissolved in 1/3 demineralised water and added to dispersion of the active pharmaceutical ingredient. Thus prepared granulation liquid was sprayed onto lactose and half of sodium corscarmellose mixture in fluid bed granulator. Microcrystalline cellulose and other half of the sodium croscaremllose were added to the obtained dry granulate and mixed in double cone mixer, finally magnesium stearate was added and mixed for a short period of time. Batch size is 1263 g.

Thus prepared granulate and pioglitazone granulate (Example 3) were compresed into bilayer tablets using rotary tabletting machine.

### Example 6

Reference product was Duetact® (TakedA) with following composition :
30 mg pioglitazone hydrochloride (as the base) with 4 mg glimepiride, povidone, croscarmellose sodium, lactose monohydrate, magnesium stearate, hydroxypropyl cellulose, polysorbate 80 and microcrystalline cellulose.

Tablets from examples 3 to 6 had their dissolutions measured under the conditions as defined in Example 1. Clearly, in a tablet of the present invention Cremophor 40 enhances dissolution when compared to sodium lauryl suplphate or a reference product containing Polysorbate 80.

## Claims

1. A pharmaceutical composition comprising a sulphonylurea-class insulin secretagogue and a surface active agent, wherein the surface active agent is obtainable by reacting castor oil or hydrogenated castor oil with ethylene oxide.

2. The pharmaceutical composition according to claim 1, wherein the surface active agent is obtainable by reacting 30 to 100 moles of ethylene oxide with 1 mole of castor oil.

3. The pharmaceutical composition according to claim 1 or 2, wherein the surface active agent is polyoxyl 40 hydrogenated castor oil (Cremophor RH 40).

4. The pharmaceutical composition according to any one of the claims 1 to 3, wherein at least 50%, preferably at least 70%, more preferably at least 90%, particularly substantially all of the sulphonylurea-class insulin secretagogue in the composition is in contact with the surface active agent.

5. The pharmaceutical composition according to any one of preceding claims, further comprising a polymer selected from a group containing cellulose derivative, polyethylene glycol, polyvinylpyrrolidone, polyvinylacetate, polyvinylalcohol, polyester, polyamide, polyanhydride, polyorthoester, polycarbonate, poly(phosphoester), poly(phosphazene), poly(iminocarbonate), mixtures and copolymers thereof, preferably is selected from the group consisting of cellulose derivative, polyethylene glycol, polyvinylpyrrolidone, polyvinylacetate, polyvinylalcohol, mixtures and copolymers thereof, more preferably is selected from hydroxypropylcellulose and hydroxypropylmethylcellulose.

6. The pharmaceutical composition according to any one of preceding claims, further comprising a disintegrant and optionally other excipients, wherein said other excipients are water soluble.

7. The pharmaceutical composition according to any one of the preceding claims, wherein the sulphonylurea-class insulin secretagogue is glimepiride, tolbutaminde, glibenclamide, gliciazide, glycopyraminde, chlorpropamide, tolazamide, acetohexamide, glipizide, glybuzole, gliquidone, glyclopyramide, glixosepid, glimidine, glypinamide, glyhexamide, glybuthiazole or a salt and/or a mixture thereof.

8. The pharmaceutical composition according to any one of the preceding claims, further comprising an active pharmaceutical ingredient selected from a thiazolidinedione class active pharmaceutical ingredient, insulin, biguanide compound, aldose reductase inhibitor, HMG-CoA reductase inhibitor, angiotensin converting enzyme inhibitor, angiotensin II antagonist, calcium antagonist and diuretic.

9. The pharmaceutical composition according to any one of the preceding claims, wherein the composition is a mixture, powder, granule, pellet, tablet, capsule, troche or combination thereof, preferably is a tablet.

10. The pharmaceutical composition according to claim 9, wherein the tablet is a bilayer tablet comprising glimepiride in a first layer and pioglitazone, preferably pioglitazone hydrochloride, in a second layer.

11. A process for preparing a pharmaceutical composition comprising a sulphonylurea-class insulin secretagogue and a surface active agent, wherein the surface active agent is obtainable by reacting castor oil with ethylene oxide, wherein the process comprises dissolving the surface active agent in a solvent, dispersing therein the sulphonylurea-class insulin secretagogue by mixing and removing the solvent.

12. The process according to claim 11, wherein mixing is preferably done by using high shear mixer.

13. The process according to claim 11 or 12, wherein the solvent is removed in a granulation process.

14. A pharmaceutical composition according to any one of the claims 1 to 10 for use as a medicine.

15. Use of a surface active agent as defined in any one of the claims 1 to 3 for enhancing dissolution of a sulphonylurea-class insulin secretagogue.
